# EUROPEAN PATENT SPECIFICATION

(11) **EP 0 898 477 B1**
(45) Date of publication and mention of the grant of the patent: **18.12.2002**
(21) Application number: 97921434.3
(22) Date of filing: 05.05.1997
(51) Int. Cl.: A61K 31/47, A61K 47/00, A61P 31/04

(54) **TOPICAL ADMINISTRATION OF PREMAFLOXACIN FOR THE TREATMENT OF SYSTEMIC BACTERIAL DISEASES**
TOPISCHE VERABREICHUNG VON PREMAFLOXACIN ZUR BEHANDLUNG VON SYSTEMISCHEN BAKTERIELLEN ERKRANKUNGEN
ADMINISTRATION TOPIQUE DE PREMAFLOXACINE DANS LE TRAITEMENT DE MALADIES BACTERIENNES SYSTEMIQUES

(30) Priority: 10.05.1996 US 644091
(43) Date of publication of application: 03.03.1999
(73) Proprietor: PHARMACIA & UPJOHN COMPANY, Kalamazoo, Michigan 49001 (US)
(72) Inventor: WATTS, Jeffrey, L., Portage, MI 49024 (US); WARDLEY, Richard, C., Hickory Corners, MI 49060 (US); STEHLE, Randall, G., Kalamazoo, MI 49009 (US)
(74) Representative: Perry, Robert Edward
(86) International application number: US9707124
(87) International publication number: WO97042954

(56) References cited:
- EP-A- 0 129 284
- WO-A-85/00108
- WO-A-92/09596
- US-A- 3 743 727
- CHEMICAL ABSTRACTS, vol. 87, no. 6, 8 August 1977 Columbus, Ohio, US; abstract no. 44205, B. BAZHDOKOV ET AL.: "DIMETHYL SULFOXIDE AS A CARRIER OF BROAD-SPECTRUM ANTIBIOTICS" XP002040745 & DERMATOL. VENEROL., vol. 16, no. 1, 1977, SOFIA, pages 33-36,
- B. IDSON: "PERCUTANEOUS ABSORPTION ENHANCERS" DRUG & COSMETIC, vol. 137, no. 1, 1985, pages 30-32, XP002040744
- CHEMICAL ABSTRACTS, vol. 122, no. 4, 23 January 1995 Columbus, Ohio, US; abstract no. 38855, J. MINGWEI ET AL.: "MANUFACTURE OF TRANSDERMAL THERAPEUTIC SYSTEM CONTAINING QUINOLONE DERIVATIVES" XP002040746 & CN 1 081 103 A (FAMING ZHUANLI SHENQING GONGKAI SHUOMINGSHU) 26 January 1994

## Description

### FIELD OF THE INVENTION

The present invention relates to a method of topically administering antimicrobial agents for the treatment of systemic bacterial diseases in mammals.

### BACKGROUND OF THE INVENTION

It has been generally accepted that intravenous infusion, intramuscular injection, subcutaneous, buccal, oral, and rectal routes are the methods for administration of a wide variety of antimicrobial agents for the treatment of systemic bacterial diseases. Due to lack of systemic level effects with antimicrobial agents administered topically, the topical administration of antimicrobial agents has been limited to the treatment of localized infections of the skin or eyes.

However, it is known that the aforementioned non-topical methods of administration for the treatment of systemic bacterial diseases have certain disadvantages. For example, buccal and rectal administration often produce discomfort and aggravation to the mammals that are treated. The intravenous, subcutaneous and intramuscular routes are not only painful, but also must be performed by trained individuals. In addition, there is a risk of needle injury, infection, and other trauma including the emotional trauma inevitably associated with injections. Oral administration, although generally acceptable, may have the disadvantages of poor absorption of the therapeutic agent from the gastrointestinal tract and/or degradation which may be caused by the acidic medium of the stomach, or causes digestive disfunction in ruminants. Furthermore, in the case of treating animals, the aforementioned methods of administration are labor and time consuming. Topical administration of antimicrobial agents would circumvent these problems by allowing a more convenient, non-invasive method for the treatment of systemic bacterial diseases.

Thus, it is desirable to have antimicrobial agents that produce systemic effects when they are topically administrated for the treatment of systemic diseases.

Premafloxacin, ciprofloxacin, enrofloxacin, danofloxacin, and their quinolone families are potent antimicrobial agents with a broad spectrum of antimicrobial activity. These quinolone families are active against a variety of human and veterinary pathogens, including both gram-positive and gram-negative bacteria. Premafloxacin and its esters are disclosed in US-A-4,665,079 and US-A-5,563,155. Ciprofloxacin and enrofloxacin are disclosed in UA-A-4,670,444. Danofloxacin is disclosed in EP-A-0215650.

Ceftifur, cefquinome and cefpodoxime are derivatives of the family of cephalosporins, which have been recognized as highly active antimicrobial agents. Ceftiofur is disclosed in US-A-4,464,367. Cefpodoxime is disclosed in US-A-4,486,425. Cefquinome is disclosed in US-A-4,754,031, EP-A-64,740 and EP-A-0074645.

Gentamicin is a aminoglycoside antibiotic complex derived from *Micromonospora purpurea* or *M*. *echinospora*. Gentamicin is effective against a wide range of aerobic gram-negative bacilli, especially the *Enterobacteriaceae* and *Pseudomonas*, and some gram-positive bacteria. Gentamicin is disclosed in, *inter alia*, US-A-3,091,572 and US-A-3,136,704.

Erythromycin is an intermediate spectrum macrolide antibiotic, produced by *Streptomyces erythreus*, effective against most gram-positive and certain gram-negative bacteria. Erythromycin is disclosed in, *inter alia*, US-A-2,653,899 and US-A-2,823,203.

### SUMMARY OF THE INVENTION

According to the present invention, a composition, for topical therapeutic use, comprises an antimicrobial agent and a carrier comprising propylene glycol (PG) and oleyl alcohol (OA) or PG and Azone, wherein the antimicrobial agent is of formula II or III 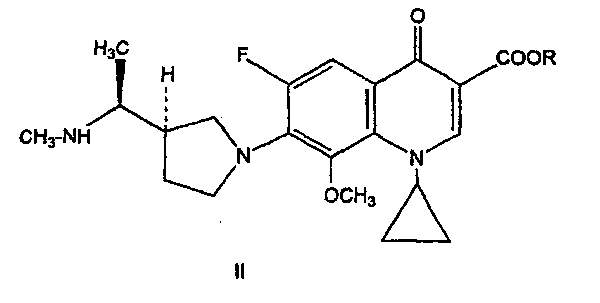 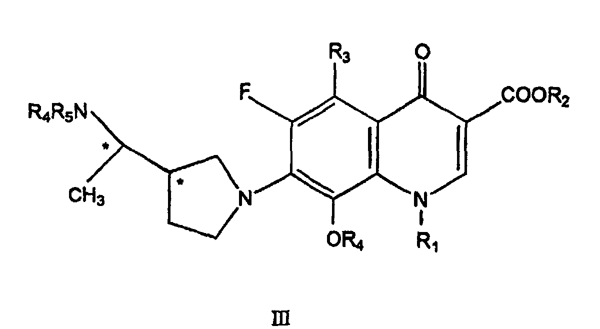 wherein R is C₁₋₁₀ alkyl, alkenyl of up to 10 C atoms, cycloalkyl of up to 6 C atoms, phenyl, benzyl or naphthyl; * denotes an asymmetric carbon atom; R₁ is ethyl, cyclopropyl or 2,4-difluorophenyl; R₂ is hydrogen, C₁₋₄ alkyl or a cation; R₃ is hydrogen, amino or methyl; and R₄ and R₅ are each independently hydrogen or methyl.

The present invention is based on the unexpected discovery that premafloxacin and its esters (of formula II) and related compounds (offormula III), when using PG/OA or PG/azone as a carrier and administered topically, are effective for the treatment of systemic bacterial infections.

### DESCRIPTION OF THE INVENTION

For the purpose of the present invention, systemic bacterial diseases means systemic bacterial infections caused by a variety of human and veterinary pathogens, including both gram-positive and gram-negative bacteria. Such diseases and conditions are well known and readily diagnosed by physicians and veterinarians of ordinary skill.

The term mammal refers to man and animals of veterinary interest. However, the invention may also be practised on other vertebrates, and on lower species comprising non-vertebrates.

Azone refers to hexadydro-1-dodecyl-2H-acepin-2-one.

Topical administration or application means the direct contact of an antimicrobial formulation with skin such as, for example, by drops, spray, paint, or pour on. The active antimicrobial formulation of the present invention is administered 1 to 5 times daily until the bacterial infection is treated.

Topical formulations of this invention may be prepared by employing conventional technique to dissolve an antimicrobial agent described above in PG and OA, or in PG and Azone. Optionally, the compositions may contain conventional stabilizers and thickening agents.

For example, about 95% of PG by volume is mixed with about 5% of OA by volume, or about 95% of PG by volume is mixed with about 5% of Azone by volume. An appropriate amount of a premafloxacin ester is weighed out and transferred to the a graduated mixing flask. The desired carrier is then added to the appropriate volume mark. The flask is sealed and the contents is mixed on a rotator until the solution is clear.

The term PG/OA carrier refers to the mixture solution of propylene glycol and oleyl alcohol. Propylene glycol and oleyl alcohol is in a ratio at least about 80% of propylene glycol by volume. The use of 95% propylene glycol and 5% oleyl alcohol achieves the best antimicrobial effect in the present invention.

The term PG/Azone carrier refers to the mixture solution of propylene glycol and Azone. Propylene glycol and oleyl alcohol is in a ratio at least about 80% of propylene glycol by volume. The use of 95% propylene glycol and 5% Azone achieves the best antimicrobial effect in the present invention.

The amount of an antimicrobial agent in a formulation may be varied or adjusted widely depending upon the solubility of the particular antimicrobial agent, the potency of the particular antimicrobial agent being used, the severity of the bacterial infection, the particular formulation, and the desired concentration. Generally, the amount of an antimicrobial agent is present in the formulation in a range between about 0.5% to about 90% by weight of the formulation, preferably between about 1% to about 25% by weight of the formulation.

In therapeutic use for treating systemic bacterial infections in mammals, an antimicrobial agent is administered topically at a dosage to obtain and maintain a concentration, of which the blood-level of active agent in the mammal being treated is antibacterial effective. Generally, the effective amount of the active agent will be in the range of about 0.1 to about 100 mg/kg, more preferably about 1.0 to about 50 mg/kg of body weight/day. It is understood that the dosages may vary depending upon the requirements of the subject being treated, the severity of the bacterial infection, and the particular antimicrobial agent being used. Also, it is to be understood that the initial dosage administered may be increased beyond the above upper level in order to rapidly achieve the desired blood-level or the initial dosage may be smaller than the optimum and the daily dosage may be progressively increased during the course of treatment depending upon the particular situation.

The present invention achieves the desired results as demonstrated in the mouse systemic protection tests. Mice that had been infected with *Pasteurella haemolytica* are given doses of a formulation of the present invention. The unexpected result of the present invention is realized by their ED₅₀ values. The unexpected results are also demonstrated by the comparison with other routes of administration, with other formulations, and with variety of antimicrobial agents.

### BIOLOGICAL TESTING

### 1. Bacteria.

The challenge organism, *Pasteurella haemolytica* UC6531, was originally isolated from a case of bovine pneumonia. The organism was kept frozen at -70 °C in trypticase soy broth containing 10% glycerol on 3 mm glass beads. Prior to use, the organism was subcultured onto a trypticase soy agar plate containing 5% sheep blood and incubated for 18-24 hours at 35 °C under aerobic conditions. A single colony was removed from the blood agar plate, inoculated in 6 ml brain-heart infusion broth (BHIB) and incubated for 6 hours at 35 °C in a 5% CO₂ atmosphere. The organism was then mixed with an equal volume of BHIB containing 2% brewer's yeast which served as the final inoculum.

### 2. Mouse Systemic Protection Tests

The P. *haemolytica* mouse systemic protection test was used to evaluate the activity of a new extended spectrum antimicrobial agents by topical administration, For this model, 21-25 day old female CD-1 mice were used. Mice were injected with approximately 100 LD₅₀ doses of the challenge organism intraperitoneal. Antimicrobial agents were administered (0.1 ml) either by subcutaneous injection, orally (subcutaneous and oral administration served as positive controls) or by topical administration. For topical administration, the antimicrobial agent was dissolved in the appropriate solution and placed in a 1 cm² area on the back. All antimicrobial agents were administered within one hour of challenge and at 24 hours intervals for the two following days for a total treatment time of three days. Ten mice were used in each treatment group at each dosage level with five, two-fold serial dilutions of the antimicrobial agent in each determination. The post-challenge effectiveness was calculated based on post-challenge day 6 and was reported as the ED₅₀, the amount of antimicrobial agent (mg/kg body weight/day) required to protect 50% of infected mice. Antimicrobial agents with lower ED₅₀ values are expected to be more effective. It is generally considered that an agent with a ED₅₀ value less than 10 will display efficacious therapeutic effects in treating systemic bacterial diseases in mammals.

The following Table reports results obtained by the topical administration of premafloxacin and its esters in formulations containing 95% propylene glycol and 5% oleyl alcohol or 5% Azone. The results are compared with topical administration of premafloxacin in 100% DMSO and oral administration of premafloxacin in aqueous solution.

| Antimicrobial Agent | Administration Route | Formulation | ED₅₀ (mg/kg/day) |
|---|---|---|---|
| Premafloxacin | Oral | Aqueous Solution | 1.6 |
| Premafloxacin | Topical | 100% DMSO | 1.7 |
| Premafloxacin | Topical | PG/OA (95:5) | 0.9 |
| Premafloxacin butyl ester | Topical | PG/OA (95:5) | 0.6 |
| Premafloxacin butyl ester | Topical | PG/Azone (95:5) | 0.3 |
| Premafloxacin ethyl ester | Topical | PG/OA (95:5) | 0.7 |
| Premafloxacin ethyl ester | Topical | PG/Azone (95:5) | 0.4 |

## Claims

1. A composition, for topical therapeutic use, comprising an antimicrobial agent and a carrier comprising propylene glycol (PG) and oleyl alcohol (OA) or PG and Azone, wherein the antimicrobial agent is of formula II or III 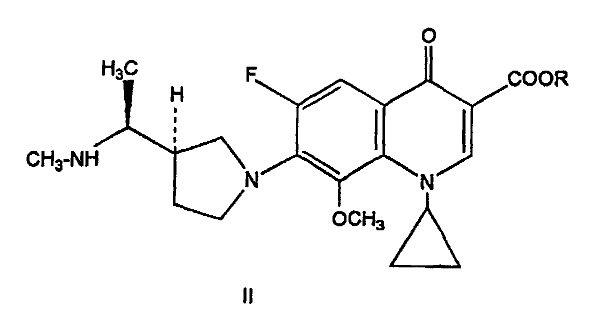 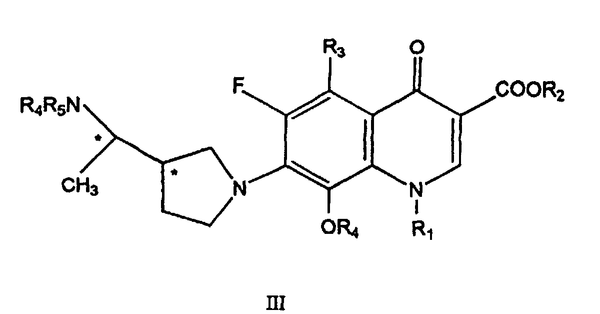 wherein R is H, C₁₋₁₀ alkyl, alkenyl of up to 10 C atoms, cycloalkyl of up to 6 C atoms, phenyl, benzyl or naphthyl; * denotes an asymmetric carbon atom; R₁ is ethyl, cyclopropyl or 2,4-difluorophenyl; R₂ is hydrogen, C₁₋₄ alkyl or a cation; R₃ is hydrogen, amino or methyl; and R₄ and R₅ are each independently hydrogen or methyl.

2. A composition according to claim 1, wherein the antimicrobial agent is premafloxacin.

3. A composition according to claim 1, wherein the antimicrobial agent is a compound of formula II.

4. A composition according to claim 3, wherein R is C₁₋₄ alkyl.

5. A composition according to claim 4, wherein R is ethyl or butyl.

6. A composition according to any preceding claim, wherein the carrier comprises, by volume, about 95% PG and about 5% OA.

7. A composition according to any of claims 1 to 5, wherein the carrier comprises, by volume, about 95% PG and about 5% Azone.

8. Use of an antimicrobial agent as defined in claim 1, for the manufacture of a topical medicament including a carrier as defined in claim 1, for the treatment or prevention of systemic or bacterial diseases.

9. The use of claim 8, wherein the antimicrobial agent is as defined in any of claims 2 to 5.

## Revendications

1. Composition, destinée à une utilisation thérapeutique topique, comprenant un agent antimicrobien et un support comprenant du propylène-glycol (PG) et de l'alcool oléylique (OA) ou du PG et de l'Azone, dans laquelle l'agent antimicrobien répond à la formule II ou III 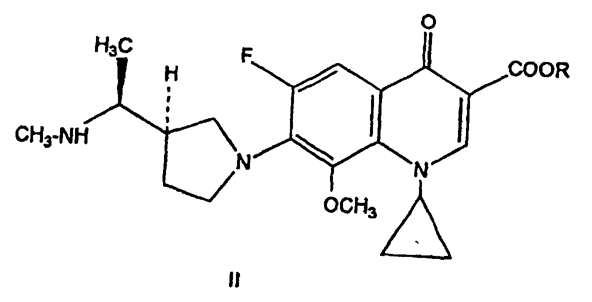 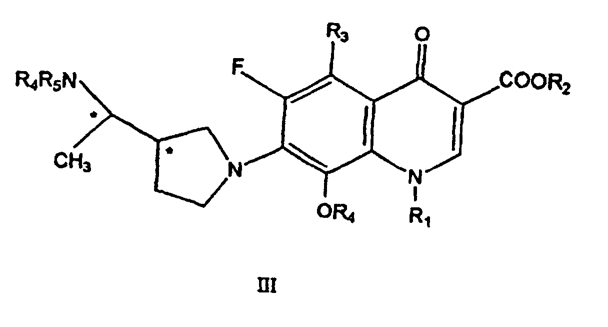 dans laquelle R représente H, un groupe alkyle en C₁ à C₁₀, alcényle ayant jusqu'à 10 atomes de carbone, cycloalkyle ayant jusqu'à 6 atomes de carbone, phényle, benzyle ou naphtyle ; le signe * désigne un atome de carbone asymétrique ; R₁ représente un groupe éthyle, cyclopropyle ou 2,4-difluorophényle ; R₂ représente l'hydrogène, un groupe alkyle en C₁ à C₄ ou un cation ; R₃ représente l'hydrogène, un groupe amino ou méthyle ; et R₄ et R₅ représentent chacun, indépendamment, l'hydrogène ou un groupe méthyle.

2. Composition suivant la revendication 1, dans laquelle l'agent antimicrobien est la prémafloxacine.

3. Composition suivant la revendication 1, dans laquelle l'agent antimicrobien est un composé de formule II.

4. Composition suivant la revendication 3, dans laquelle R représente un groupe alkyle en C₁ à C₄.

5. Composition suivant la revendication 4, dans laquelle R représente un groupe éthyle ou butyle.

6. Composition suivant l'une quelconque des revendications précédentes, dans laquelle le. support comprend, en volume, environ 95 % de PG et environ 5 % de OA.

7. Composition suivant l'une quelconque des revendications 1 à 5, dans laquelle le support comprend, en volume, environ 95 % de PG et environ 5 % d'Azone.

8. Utilisation d'un agent antimicrobien répondant à la définition suivant la revendication 1, pour la production d'un médicament topique comprenant un support répondant à la définition suivant la revendication 1, pour le traitement ou la prévention de maladies systémiques ou bactériennes.

9. Utilisation suivant la revendication 8, dans laquelle l'agent antimicrobien répond à la définition suivant l'une quelconque des revendications 2 à 5.

## Patentansprüche

1. Zusammensetzung zur topischen therapeutischen Verwendung, die ein antimikrobielles Mittel und einen Träger, der Propylenglykol (PG) und Oleylalkohol (OA) oder PG und Azon umfasst, umfasst, wobei das antimikrobielle Mittel die Formel II oder III aufweist, 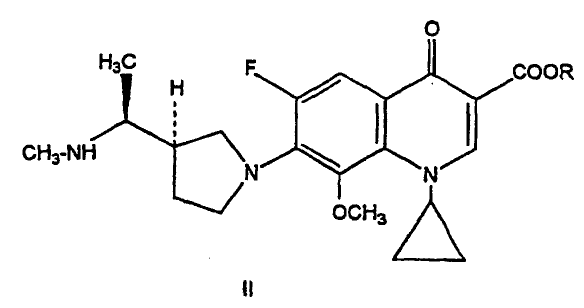 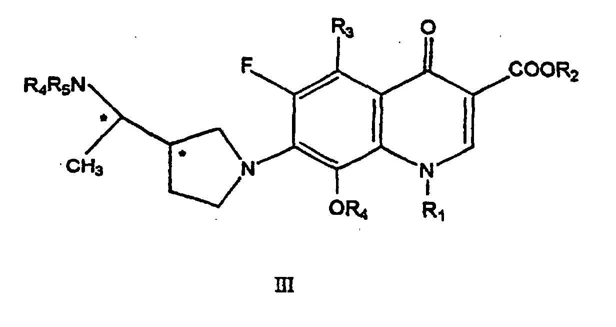 worin R H, C₁₋₁₀-Alkyl, Alkenyl mit bis zu 10 C-Atomen, Cycloalkyl mit bis zu 6 C-Atomen, Phenyl, Benzyl oder Naphthyl ist, * ein asymmetrisches Kohlenstoffatom bezeichnet; R₁ Ethyl, Cyclopropyl oder 2,4-Difluorphenyl ist; R₂ Wasserstoff, C₁₋₄-Alkyl oder ein Kation ist; R₃ Wasserstoff, Amino oder Methyl ist; und R₄ und R₅ unabhängig voneinander Wasserstoff oder Methyl sind.

2. Zusammensetzung nach Anspruch 1, worin das antimikrobielle Mittel Premafloxacin ist.

3. Zusammensetzung nach Anspruch 1, worin das antimikrobielle Mittel eine Verbindung der Formel II ist.

4. Zusammensetzung nach Anspruch 3, worin R C₁₋₄-Alkyl ist.

5. Zusammensetzung nach Anspruch 4, worin R Ethyl oder Butyl ist.

6. Zusammensetzung nach einem der vorhergehenden Ansprüche, worin der Träger; bezogen auf das Volumen, etwa 95 % PG und etwa 5 % OA umfasst.

7. Zusammensetzung nach einem der Ansprüche 1 bis 5, worin der Träger, bezogen auf das Volumen, etwa 95 % PG und etwa 5 % Azon umfasst.

8. Verwendung eines antimikrobiellen Mittels gemäß der Definition in Anspruch 1 zur Herstellung eines topischen Medikaments, das einen Träger gemäß der Definition in Anspruch 1 umfasst, zur Behandlung oder Prophylaxe von systemischen oder bakteriellen Erkrankungen.

9. Verwendung nach Anspruch 8, wobei das antimikrobielle Mittel wie in einem der Ansprüche 2 bis 5 definiert ist.
